# EUROPÄISCHE PATENTSCHRIFT

(11) **EP 2 004 116 B1**
(45) Veröffentlichungstag und Bekanntmachung des Hinweises auf die Patenterteilung: **15.06.2011**
(21) Anmeldenummer: 07724155.2
(22) Anmeldetag: 11.04.2007
(51) Int. Cl.: A61F 13/00

(54) **PRIMÄRVERBAND**
PRIMARY DRESSING
PANSEMENT PRIMAIRE

(30) Priorität: 12.04.2006 DE 102006017194
(43) Veröffentlichungstag der Anmeldung: 24.12.2008
(73) Patentinhaber: Riesinger, Birgit, 48346 Ostbevern (DE)
(72) Erfinder: Riesinger, Birgit, 48346 Ostbevern (DE)
(74) Vertreter: Michalski, Stefan
(86) Internationale Anmeldenummer: PCT/EP2007/003214
(87) Internationale Veröffentlichungsnummer: WO 2007/118652

(56) Entgegenhaltungen:
- EP-A- 0 934 736
- EP-A1- 0 302 611
- WO-A-00/16726
- WO-A-00/59436
- WO-A-97/03795
- DE-U1-202004 017 052
- US-A- 5 620 771

## Beschreibung

Die Erfindung betrifft einen flüssigkeitsdurchlässigen Primärverband in Form eines schmiegsamen thermoplastischen Materialabschnittes, der aufweist:
- eine erste, glatte Oberfläche,
- eine der glatten Oberfläche abgewandte, zweite Oberfläche des Materialabschnittes,
- eine Vielzahl von dreidimensionalen Perforationen, deren Wände von der ersten, glatten Oberfläche beginnend jeweils in einen Randüberstand mit freier Kante auslaufen und der zweiten Oberfläche einen rauen Griff verleihen.

Derartige flüssigkeitsdurchlässigen, thermoplastischen Materialabschnitte werden als sogenannte Topsheets in Hygienebereich, vornehmlich in Damenhygiene, als Krankenhausunterlagen oder bei Babywindeln eingesetzt. Der US 30 54 148 ist ein perforiertes Folienmaterial zu entnehmen, welches eine Vielzahl von dreidimensional ausgebildeten Perforationen aufweist. Die Perforationen werden mit Hilfe eines an einer Trommel angeordneten Lochsiebs im thermisch unterstützten Vakuumverfahren hergestellt. Dementsprechend weist das fertige Folienmaterial eine glatte und eine raue, durch die abgewinkelten Wandungen der Perforationen gebildete Oberfläche auf.

Die WO 00/59436 A1 zeigt ein vorzugsweise in beide Richtungen flüssigkeitsundurchlässiges Backsheet wie es bei vom Körper abgewandten Folienmaterialien, z.B. bei Windeln oder Damenbinden, als luft- aber nicht flüssigkeitsdurchlässiges Folienmaterial eingesetzt wird. Das Plastikgewebe besitzt Perforationen, die zu Kapillaren geformt sind und deren Wände von der ersten, glatten Oberfläche beginnend jeweils in einen Randüberstand mit freier Kante auslaufen und so der zweiten Oberfläche einen rauen Griff verleihen. So entsteht eine atmungsaktive Schutzfolie, die sich jedoch nicht zum Einsatz als Primärverband eignet.

Aus WO 97/03795 A1 ist eine flüssigkeitsdurchlässige Plastikfolie in Form eines schmiegsamen thermoplastischen Materialabschnitts mit einer Vielzahl an Kapillaren bekannt. Da die Kapillaren weit über die Oberfläche der Folie hinausragen, bekommt das Material eine raue Griffigkeit. Durch die langen Kapillaren eignet sich die Folie insbesondere zum Durchleiten großer Flüssigkeitsmengen in darunterliegende Absorptionsschichten wie es z.B. bei Krankenhausunterlagen, die zum Schutz der Bettwäsche nach Operationen eingesetzt werden, der Fall ist.

Die WO 00/16726 A1 zeigt einen lediglich gasdurchlässigen Film mit eine ersten, glatten Oberfläche und einer der glatten Oberfläche abgewandten, zweiten Oberfläche, sowie eine Vielzahl an dreidimensionalen Perforationen, deren Ränder an der Ausgangsöffnung in unterschiedlicher Höhe enden und somit einen Randüberstand bilden. Die Wände der Perforationen beginnen an der ersten Oberfläche und erstrecken sich im Verhältnis zur ersten Oberfläche nicht-vertikal weit über die zweite Oberfläche hinaus.

Weiterhin ist aus EP 0 081 988 B1 ein Primärverband bekannt, der ebenfalls Perforationen aufweist. Der Schrift sind keine Einzelheiten bezüglich Oberflächenbeschaffenheit des den Primärverband bildenden Materialabschnittes zu entnehmen. Es ist keine raue und keine glatte Oberfläche beschrieben worden. Die Peripherie des Primärverbandes ist frei von Perforationen. Eine solche Ausführung des Primärverbandes ist teuer. Außerdem ist ein Sekundärverband (Wundkissen) über eine weitere Klebeschicht sandwichartig mit dem Primärverband verbunden.

Die Aufgabe der Erfindung ist, den Einsatzbereich der Materialabschnitte der eingangs genannten Art um die Wundbehandlung zu erweitern. Insbesondere soll der Materialabschnitt zur Wundabdeckung als Primärverband dienen, auf den saugfähige Sekundärverbände kleblos auflegbar sind und der den Verklebungen und Adhäsionen mit dem Wundexsudat entgegenwirkt. Es soll ein nahezu idealerweise punktuell aufliegendes, hypoallergenes, kostengünstiges Wundkontaktgitter zur Verfügung stehen, das an einen auswechselbaren, nachgeschalteten Absorptionskörper anschließbar ist und dabei auch unter Druck aufgrund eines ausgewählten Flächenverhältnisses zwischen Material und Lücke den Durchtritt von Wundflüssigkeiten zu garantieren, wobei durch die Wahl der trichterförmigen Materialumlenkungen das Folienmaterial eine Orientierung in eine glatte, der empfindlichen Wunde entsprechende Fläche und eine raue Seite bestimmt wird.

Ein Primärverband gemäß Erfindung, der zur Wundabdeckung unter Vermeidung von Verklebungen und Adhäsionen mit dem Wundexsudat dient, auf den wenigstens ein saugfähiger Sekundärverband auflegbar ist, umfasst:
- einen schmiegsamen Materialabschnitt aus einem Thermoplasten, welcher Materialabschnitt eine erste, glatte Oberfläche und eine der glatten Oberfläche abgewandte und von dieser beanstandete, zweite Oberfläche aufweist,
- eine Vielzahl von dreidimensionalen Perforationen, deren Wände von der ersten, glatten Oberfläche beginnend jeweils in einen Randüberstand mit freier Kante auslaufen und der zweiten Oberfläche einen rauen Griff verleihen, wobei wenigstens eine der freien Kanten in einen etwa senkrecht zu einer Perforationsachse (A) abgewinkelten Abschnitt übergeht.

Dabei sollen die im Wesentlichen konisch ausgebildeten Wände der Perforationen klein genug sein für eine durchgängige Meniskusbildung von Flüssigkeiten mit spezifischem Gewicht zwischen 0,9 und 1,2. Das spezifische Gewicht von 0,9 bis 1,2 entspricht dem flüssigen Wundexsudat. Unter dem Begriff Meniskus wird im vorliegenden Fall eine konvexe oder konkave Oberfläche der sich kapillar bewegenden Wundflüssigkeit, die auf die Wechselwirkung der Wundflüssigkeit mit den Oberflächen des Primärverbandes und der jeweiligen Wunde zurückgeht.

Die abgewinkelten, an der rauen Oberfläche befindlichen Abschnitte bewirken eine wünschenswerte Verminderung des Rückflusses bereits eingetretener Wundflüssigkeiten.

Der Materialabschnitt soll als Additiv zu einem Wundverband verstanden werden, welches im Direktkontakt mit der Wunde Einsatz findet und auf Grund seines dreidimensionalen Form nicht auf seiner gesamten Fläche Kontakt zur Wunde hat, wobei dieses Additiv keine absorbierende Funktion oder Vorrichtung hat, sondern im Gegenteil mit anderen, als Sekundärverband fungierenden Produkten auswechselbar kombiniert werden soll. Er bildet damit im Sinne der Erfindung einen Primärverband. Das Additiv bzw. der Primärverband soll Verwendung finden bei akuten und chronischen Wunden, iatrogenen Durchtrennungsstellen der Haut, Verbrennungswunden, nässenden entzündlichen Prozessen der Haut oder exulzerierenden Prozessen neoplastischer Genese, nässenden Infektionen, Fisteln, Postoperativen Drainagen, Stomata, atopisch veränderlichen Arealen der Haut, gelenknahen Hautumschlagsfalten wie Achsel- oder Leistenhaut, Schleimhautoberflächen des Menschen und des Tieres sowie in Verbindung mit anderen Verbandstoffen, die lokaltherapeutischen Effekt haben und bei anderen Anwendungen, bei denen eine atraumatische Wundauflage indiziert ist.

Die Herstellung einer dreidimensionalen Folie der hier zu beschreibenden Art bedingt, den genannten Zielsetzungen folgend, die Entstehung einer glatten und einer rauen Oberfläche, wobei beide Oberflächen eine wundheilungsförderliche Eigenschaft aufweisen können. Die glatte Oberfläche schützt die Wunde vor Irritation und unerwünschten Einflüssen eines Sekundärverbandes, während die raue dies auch durchführt, hingegen aber aktiv bei Bewegung an der Wunde scheuert und somit einen erwünschten chemotaktischen Reiz auf die Gewebeneubildung bedeuten kann.

Die Oberfläche des Materialabschnittes ist vorzugsweise gerade und plan ausgeformt, wobei die Perforationen bzw. Löcher durch ausschussartige Materialumlenkungen begrenzt werden können. Die entstehenden Krater, Trichter oder auch Füße haben vorzugsweise die gleiche Tiefe, so dass eine Auflagefläche entsteht, die allein aus den der ehemaligen Oberfläche abgewandten Rändern der ehemals planen, nun in bis zu 30 bis 179 Grad umgelenkten Abschnitten bestehen kann.

Die Perforationen selbst können polygonal, rund, oval, drei- oder mehreckig oder auch ohne vorgegebene Struktur sein, bilden jedoch bevorzugt Strassen aus homogenen Lochgeometrien.

An den Rändern der der ehemaligen Oberfläche abgewandten Krater können fertigungsbedingt ebenfalls plane Reste stehen, die nicht oder nur teils ausgestanzt wurden. Ziel kann es jedoch sein, die Enden der Trichter ohne zweite oder gar erste unvollständige Umlenkung in die zur planen Oberfläche etwa parallel verlaufenden Reste auszuformen, so dass eine Gitterstruktur angestrebt wird, die in ihrer Gesamtheit plan, aber mit etwa rechtwinklig in nur eine Richtung abstehenden, sich wiederholenden und gleichtiefen Kratern vorliegt.

Es sind auch Ausführungsformen denkbar, die an der unteren Begrenzung der Trichter über parallel zur Flachseite verlaufende, kleinflächige, plane Materialabschnitte verfügt, die beispielsweise dadurch entstehen, dass die Löcher zweckmäßigerweise nicht vollständig ausgestanzt sind. Diese Abschnitte tragen trotz ihrer möglicherweise nur geringen Fläche dazu bei, dass aufgenommenes Wundexsudat nur erschwert in Richtung der Wunde zurückfließen kann. Dieser Effekt wird zwar im Wesentlichen durch die Ausrichtung der Trichter und Krater erzeugt, kann aber durch diese Flächen unterstützt werden.

Der Materialabschnitt soll hart genug sein, die Reißfestigkeit zu sichern, insbesondere wegen der vorhandenen Perforationen, zum anderen jedoch soll weich genug sein, um Brüchigkeit zu vermeiden und Flexibilität zu schaffen.

Es kann sinnvoll sein, Löcher unterschiedlicher Geometrie anzustreben. Denkbar ist die Kombination von runden und länglichen Perforationen, da hierdurch zusätzliche Sicherheit gewonnen wird, auch unter Druck flächenhafte Durchtrittsgebiete zu erlangen.

Die strassenförmige Ausrichtung der Perforationen kann sinnvoll sein, um inhomogene Elastizitätsunterschiede zu vermeiden. Diese strassenförmige Ausrichtung kann dazu führen, dass richtungsabhängige Unterschiede in der Reißfestigkeit entstehen, so dass der Materialabschnitt quer zur Strasse eine höhere oder niedrigere Festigkeit aufweisen kann.

Innerhalb der Strasse kann die Lokalisation der Löcher versetzt stattfinden, so dass eine ziehharmonikaartige Platzierung von runden Perforationen eingerahmt sein kann von Strassen von länglichen Löchern.

Als Material kann Polyethylen in Frage kommen, insbesondere UV-instabiles PE sehr geringer Dichte (ULDPE, ultra density polyethlene), wobei auch andere Kunststoffe, Naturstoffe oder Verbindungen beider Arten gewählt werden können. Sie können Trägersubstanz sein für Pharmaka wie z. B. Antibiotika, Wachstumsfaktoren, Entzündungshemmer (NSAID, Steroide oder andere Gruppen). Sie können (un-) mittelbar verbunden sein mit Carboxymethylcellulose, Metallpartikeln, insbesondere Nanometallen, Vermischungen mit Honig und dessen Derivaten, Aloe vera, Hydrofasern, Desinfektionsmitteln, Hydrogelen, Enzymen, Fetten, Vitaminen, Mineralien, Collagen, Antikörpern, superabsorbierenden Granulaten oder ähnlichen Lokaltherapeutika. In Verbindung mit dem Wunddistanzgitter ist es auch denkbar, dass die superabsorbierenden Granulate wie ein Depot mit Lösungen von Pharmaka getränkt vorliegen, so dass die Wirkstoffe über eine kontinuierliche Abgabe in die Wundregion appliziert werden können; je nach Resorption wäre über diesen Mechanismus auch eine systemisch wirkende Applikation denkbar.

Als Naturstoff kann beispielsweise hydrophob ausgerüstete Baumwolle oder Seide zum Einsatz kommen. Es ist auch eine künstliche Seide bzw. Spinnenseide denkbar.

Das biegsame Folienmaterial kann rückstandslos entnehmbar sein, nachdem es möglicherweise mehrere Tage auf der Wunde aufgelegen hat. Da es sodann allein zunächst appliziert worden ist, um danach einen Sekundärverband von der Wunde zu beabstanden, der möglicherweise auf Grund stärkerer Exsudation mehrfach gewechselt wurde, wurde es möglicherweise mit mehreren Absorptionskörpern chronologisch nacheinander versehen.

Das Folienmaterial kann über eine Beschichtung verfügen, die seine Adhäsion weiterhin reduziert. Coatingverfahren unterschiedlicher Art sind bereits ausgedehnt im Stand der Technik zu Folienproduktionen zu finden. Vorzugsweise beeinflusst diese Beschichtung die Flexibilität des Produktes nicht, da sich das Folienmaterial der Wundoberfläche nahezu parallel anpassen soll. Hierbei kann es wünschenswert sein, dass wenigstens Teile des Materialabschnittes auf gesunder Haut liegen, da sie die Wundfläche überragen. Teflon, Fette, silikonisierte oder mit Hydrokolloiden versehene Zusätze bieten sich an.

Es kann auch vorgesehen sein, den Primärverband geordnet oder chaotisch zu falten, um es in Form eines Wundfüllers zu verwenden oder in tunnelgebenden Taschen unter der Haut einzusetzen.

Rein geometrisch bilden die Ränder der Perforationen die Materialteile des Wunddistanzgitters, die von der Wundfläche am weitesten entfernt liegen, wenn die glatte Seite zur Wundoberfläche zeigt. Diese Applikation wird, verglichen mit der Anbringung der rauen Seite zur Wunde, den häufigsten Einsatz finden. Denn dieses bestimmt seine Eigenschaft, im Sinne einer atraumatischen Wundauflage von der Wundoberfläche abgelöst werden zu können, ohne zu Blutungen, Schmerzen oder Lösungsprozessen von verklebten Oberflächen zu führen.

Der Primärverband, im Weiteren Wunddistanzgitter genannt, liegt, auf seiner Flachseite gelegen, entweder auf den planen Flächen zwischen den Löchern der glatten Oberfläche oder auf den rauen Begrenzungen der Perforationen, da der Übergang von dem Gittermaterial zu den Löchern die Auflagefläche der rauen Oberfläche bestimmt. So kann dieses Wunddistanzgitter in zwei Orientierungen mit zwei sehr unterschiedlichen Funktionen Wundkontakt haben.

Der Einsatz dieses Wunddistanzgitters führt zur Reinhaltung von mikroperforierten Oberflächen des Sekundärverbandes, da grobe Verunreinigungen wie Fibrinbeläge, Verkrustungen oder putride Prozesse nicht direkt in die äußere Hülle des sekundär applizierten Saugkörpers gelangen, sondern im Wundgrund bleiben, ohne die Poren des Sekundärverbandes zu verstopfen. Durch diesen Effekt hält das Wunddistanzgitter bei verschmutzten Wunden die Funktion des Sekundärverbandes aufrecht und verlängert seine Applikationsdauer oder ermöglicht die Aufnahme seiner Funktion, da die o. g. Verunreinigungen an den Flächen des Wunddistanzgitters verbleiben, ohne die Löcher zu verlegen.

Die Anzahl von runden Löchern kann größer als die der ovalen Löcher, vorzugsweise 2mal größer sein. Der Durchmesser der rundlichen Löcher beträgt vorzugsweise 650 bis 800 um; die Länge der länglichen Löcher beträgt vorzugsweise 900 bis 1200 µm bei einer Breite von etwa 700 µm. Die Löcher bilden vorzugsweise 25% der Gesamtfläche in Draufsicht auf die Flachseite des Wunddistanzgitters.

Der Primärverband kann seine bevorzugte Anwendung bei Kompressionstherapien der chronischen Wunde wie z.B. Ulcus cruris oder aber der Unterdrucktherapie finden, bei der Elemente des Verbandes unter Erzeugung von subatmosphärischen Druckverhältnissen in die Wundregion gepresst werden.

Für die Verwendung als Wunddistanzgitter ist es angebracht, das Produkt zu sterilisieren und in eine ebenfalls sterilisierte, beutelartige Verpackung zu platzieren.

Die Erfindung wird nachfolgend anhand der Zeichnung erläutert. Die Figuren zeigen:
- Figuren 1a,1b,1c: ein Wunddistanzgitter mit runden und ovalen bzw. länglichen Perforationen, in Draufsicht auf seine glatte und raue Oberfläche;
- Fig. 2: einen Schnitt A-A gemäß Fig. 1a;
- Figuren 3a, 3b: eine konische Perforation in einer stark vergrößerten Darstellung;
- Fig. 4: eine andere Ausführungsform des Wund- distanzgitters, in Draufsicht auf seine Unterseite;
- Fig. 5: einen Schnitt B-B gemäß Fig. 4;
- Fig. 6: ein auf eine Wunde aufgelegtes Wund- distanzgitter mit Umschlagfalte, in einer schematischen Darstellung;
- Fig. 7: eine aus dem Material des Wunddis- tanzgitters hergestellte Tasche, in einer schematischen Seitenansicht;
- Fig. 8: das peripher mit einem Sachet ver- klebtes Wunddistanzgitter, ebenfalls in einer schematischen Seitenansicht;
- Fig. 9: Anordnung eines Sekundärverbandes und Wunddistanzgitters an einer Wunde;
- Fig. 10: ein Wunddistanzgitter gemäß Fig. 4 mit Abziehfolie;
- Fig. 11: einen Verpackungsbeutel mit darin un- tergebrachten Wunddistanzgitter; und
- Figuren 12 bis 14: die raue Oberfläche des Wunddistanz- gitters mit pyramidförmigen Perfora- tionen.

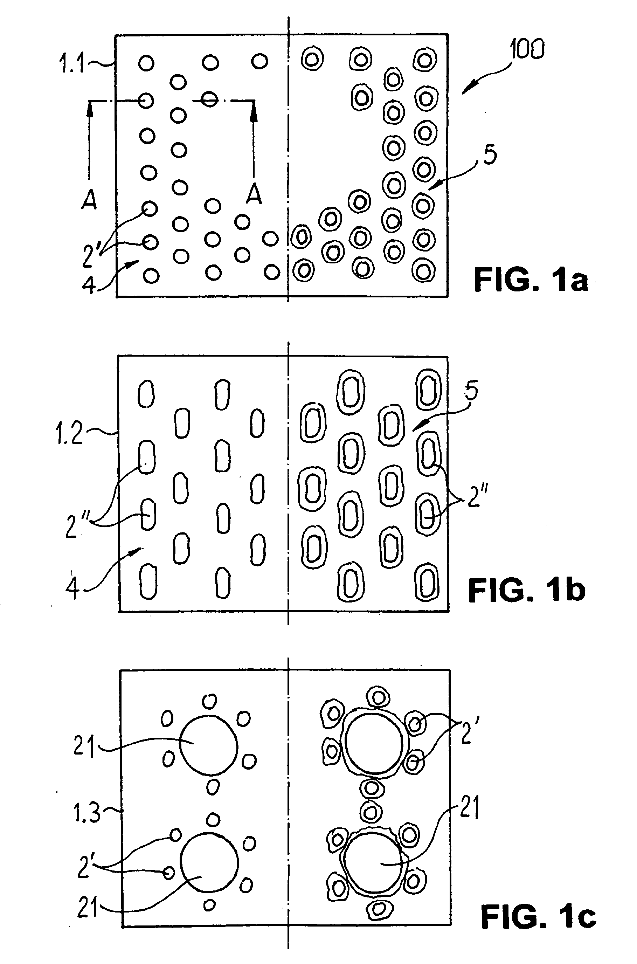

In Fig. 1a ist ein Primärverband 100 in Form eines rechteckigen, schmiegsamen Materialabschnittes 1.1 aus UVinstabilem Polyethylen von einer sehr geringen Dichte dargestellt. Die Dichte liegt im vorliegenden Fall zwischen 0,890 g/cm³ und 0,915 g/cm³.

Die linke Seite der Fig. 1a zeigt eine glatte Oberfläche 4, dagegen die rechte Seite eine raue Oberfläche 5 des Materialabschnittes 1.

Im Weiteren wird der Begriff "Materialabschnitt" durch "Wunddistanzgitter" ersetzt.

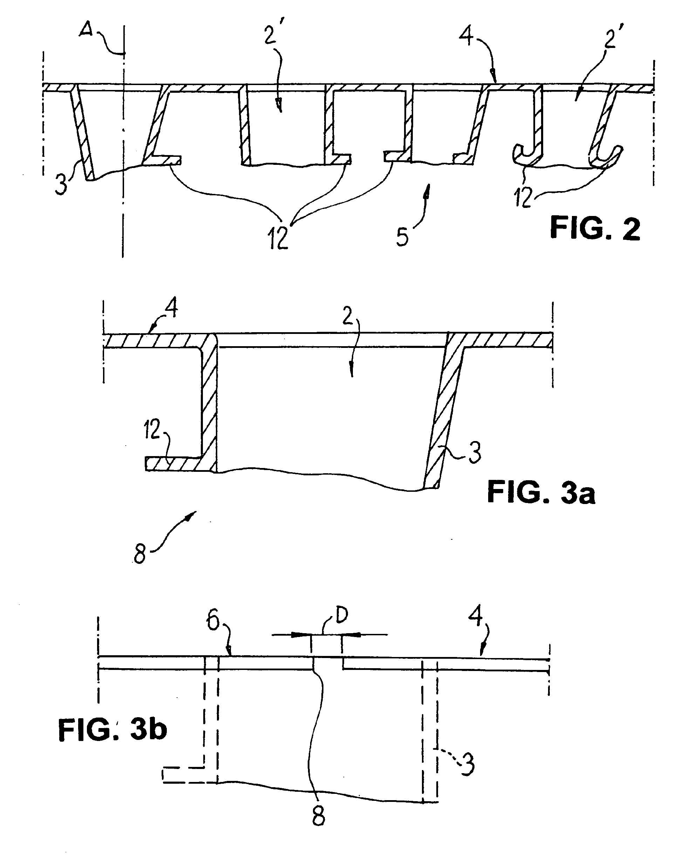

Das Wunddistanzgitter 1.1 weist eine Vielzahl von runden Perforationen 2' auf, die gemäß Fig. 2 konisch ausgebildete Wandungen 3 besitzen, welche wiederum unregelmäßig in etwa senkrecht zu einer Perforationsachse A gerichtete, fetzenartige Abschnitte 12 auslaufen. Die Abschnitte 12 können auch nach innen oder nach außen umgekrempelt sein, wie es die rechte Seite der Fig. 2 zeigt. Dies kann durch Verwendung eines warmen Luftstroms, beispielsweise mit Hilfe einer Warmluftdüse, eines Föns oder dgl. erfolgen. Der beschriebene Aufbau der Perforationen 2' trägt dazu bei, dass das aufgenommene Wundexsudat nur erschwert in Richtung der Wunde zurückfließen kann.

Bei dem in Fig. 1b gezeigten Primärverband handelt es sich um ein ähnliches Wunddistanzgitter 1.2, das eine Vielzahl von ovalen Perforationen 2" aufweist.

Gemäß Fig. 1c sind am Wunddistanzgitter 1.3 ovale Löcher 21 eingebracht, die ebenfalls in abgewinkelte Wände 3 übergehen und jeweils von einer Mehrzahl der runden Perforationen 2' umgeben sind. In vorliegendem Fall sind um das Loch 21 herum sechs runde Perforationen 2' eingebracht, jedoch ihr Anzahl kann beliebig sein.

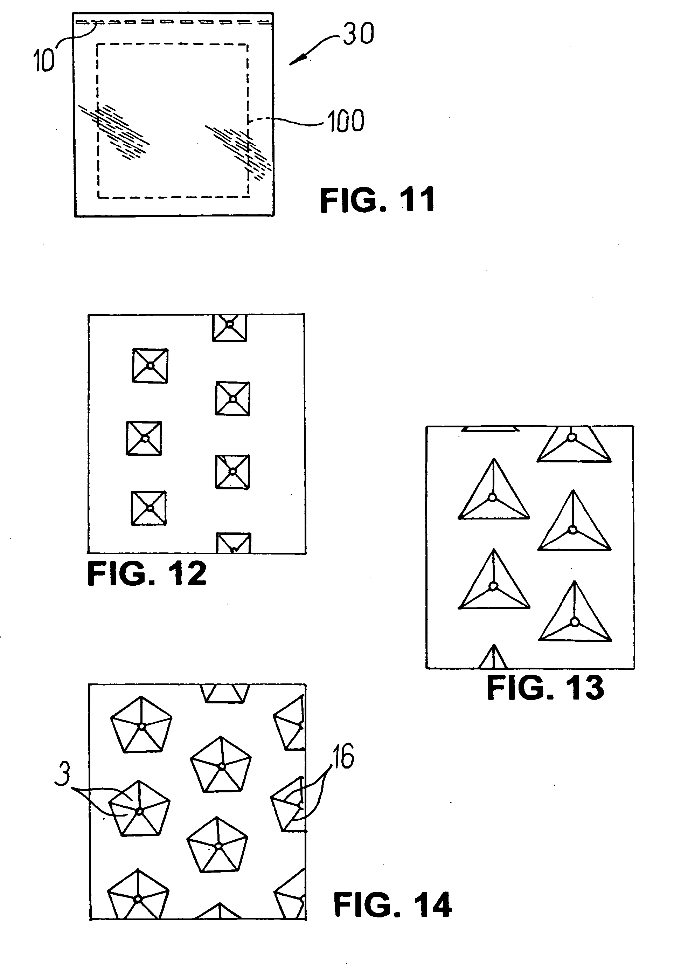

Gemäß den Figuren 12, 13 und 14 liegen die Perforationen in drei-, vier- und fünfeckigen Pyramidenformen vor. Die Wandungen der Pyramiden sind segmentiert, d. h. voneinander mit Stanzlinien 16 getrennt.

Die Fig. 3a zeigt ein vergrößertes Detail der Perforation 2' mit der konisch verlaufenden Wand 3 und mit einem nach außen abstehenden Abschnitt 12. Nach dem Ausglätten der Perforation, beispielsweise mit Hilfe eines eisenbügelartigen Werkzeuges unter Wärmezufuhr ergibt sich eine plane Fläche 6 (vgl. Fig. 3b), wobei die Perforation verschwindet nicht, sondern verkleinert sich zu einer Öffnung von einem auf der Figur gezeigten Durchmesser D, der wesentlich kleiner als die ursprüngliche Perforation 2' ist.

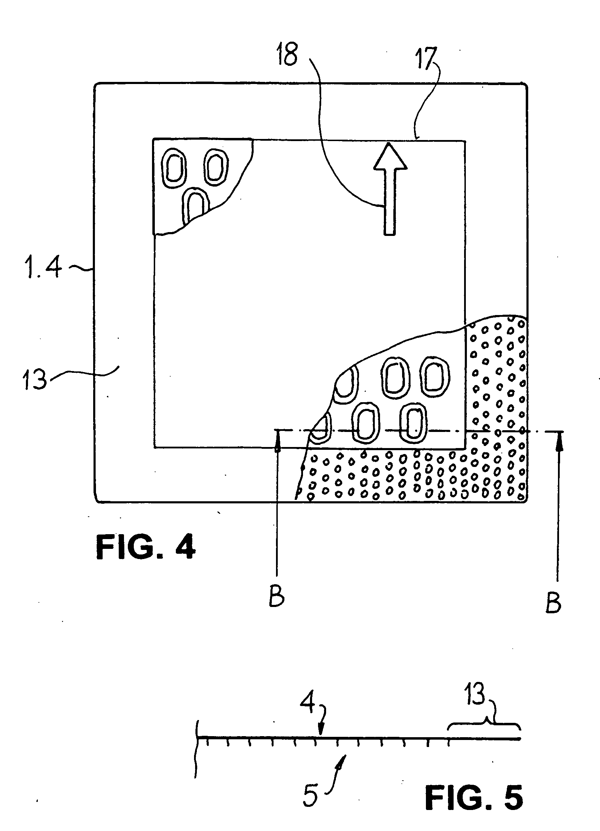

Dieses Glätten von dreidimensionalen Perforationen zu einer planen Fläche wird nun zur Erzielung einer beidseitig glatten Peripherie 13 an einem in Figuren 4 und 5 gezeigten Wunddistanzgitter 1.4 ausgenutzt. Eine am Wunddistanzgitter eingebrachte, umlaufende Markierung 17 grenzt das mittlere Feld, d. h. die raue Oberfläche 5 von der glatten Peripherie 13 optisch ab. Eine weitere Markierung 17 in Pfeilform zeigt die reißfeste Richtung.

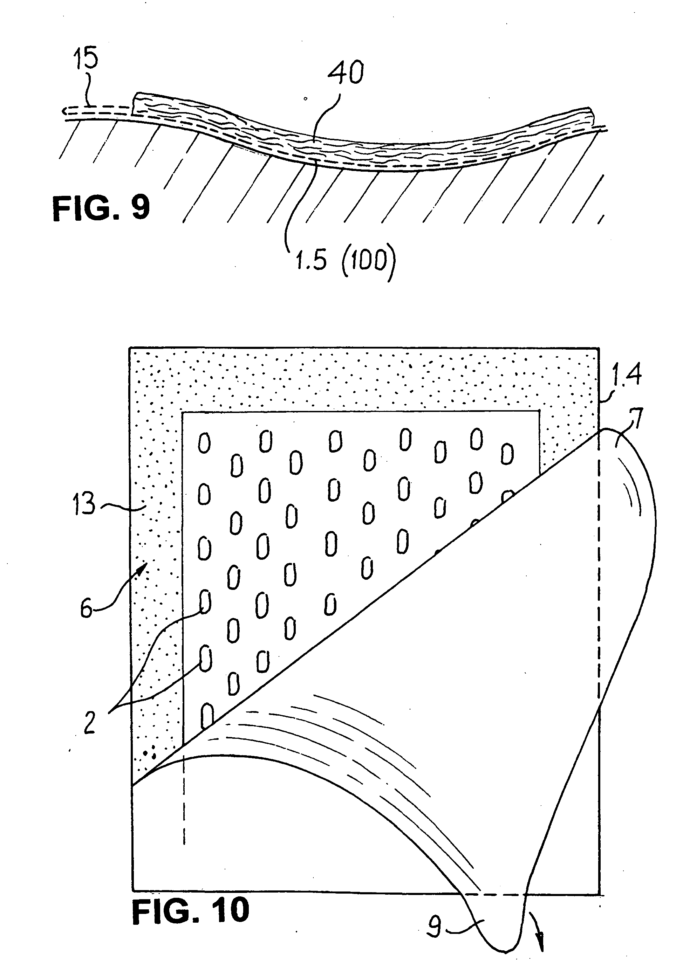

Die Fig. 10 zeigt ebenfalls das Wunddistanzgitter 1.4, jedoch mit einer peripheren Klebfläche 6, die mit der glatten Peripherie 13 deckungsgleich ist. Die Klebfläche 6 ist mit einer abziehbaren Schutzfolie 7 mit Abziehlasche 9 versehen.

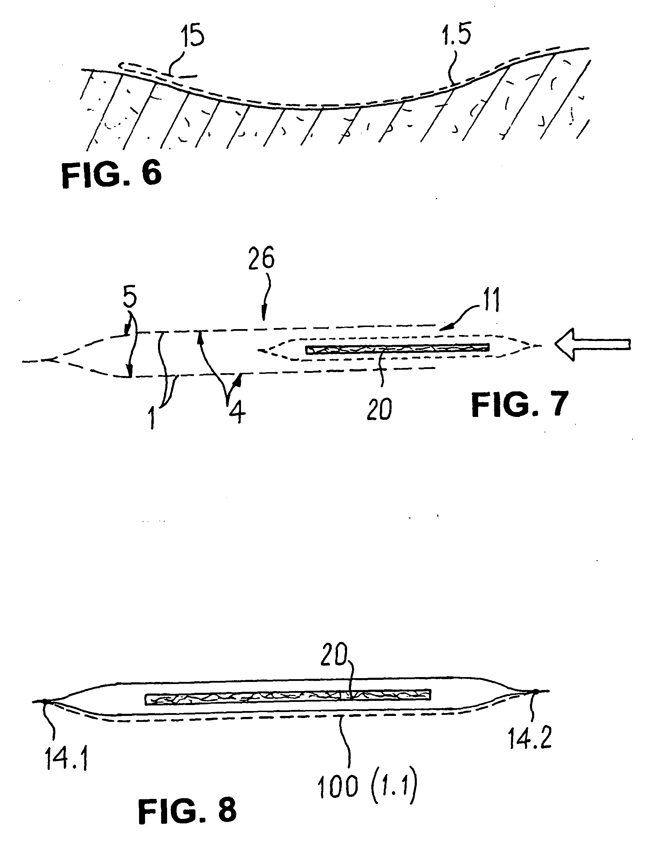

Ein in Figuren 6 und 9 dargestelltes Wunddistanzgitter 1.5 weist eine Umschlagfalte 15 auf, die ein vereinfachtes Herausnehmen sowohl des Wunddistanzgitters als auch eines aufliegenden Sekundärverbandes 40 aus der Wunde erlaubt. In besonderen Fällen kann die Umschlagfalte 15 flächenmäßig dem Wunddistanzgitter 1.5 gleichen.

Eine abweichende Ausführungsform des Wunddistanzgitters ist eine aus demselben Material gefertigte Tasche 26, die eine Öffnung 11 zum Einschieben und Herausnehmen eines umhüllten Absorptionskörpers 20 aufweist (vgl. Fig. 7). Der Absorptionskörper 20 wird von der Anmelderin unter Markennamen SORBION SACHET hergestellt. Die besagte Tasche 26 besteht aus zwei aufeinander liegenden und an ihrem Umfang, ausgenommen Öffnung 11, miteinander verschweißten Materialabschnitten 1 (Wunddistanzgitter). Die Tasche 26 weist im vorliegenden Fall zwei glatte Oberflächen 4 auf, von denen die eine ins Tascheninnere und die zweite nach außen gerichtet ist. Dementsprechend weist die Tasche 26 zwei rauhe, ebenfalls ins Innere der Tasche und nach außen zeigende Oberflächen 5 auf. In zwei weiteren, nicht dargestellten Ausführungsbeispielen hat die Tasche zwei nach innen bzw. nach außen gerichtete gleiche Oberflächen 4 bzw. 5.

Die Fig. 8 zeigt das am umhüllten Absorptionskörper 20 befestigte Wunddistanzgitter 1.1. Befestigt ist das Wunddistanzgitter 1.1 mittels zwei sich gegenüber liegender Schweißnähte 14.1, 14.2. Die Schweißnähte 14.1, 14.2 sind in Fig. 7 als Punkte sichtbar.

Gemäß Fig. 11 ist der sterilisierte Primärverband 100 (Wunddistanzgitter) in einem ebenso mittels Ethylenoxid sterilisierten, flachen Verpackungsbeutel 30 untergebracht. Der Verpackungsbeutel 30 ist mit einem hermetischen Leistenverschluss 10 versehen. Anstelle des Leistenverschlusses kann eine Markierungslinie vorliegen, falls der Verpackungsbeutel zusammengeschweißt ist.

### Bezugszeichenliste:

- 1: Materialabschnitt
- 2'; 2 ": Perforationen
- 3: Wand
- 4; 5: Oberfläche
- 6: Klebfläche
- 7: Schutzfolie
- 8: Kante
- 9: Abziehlasche
- 10: Leistenverschluss
- 11: Öffnung
- 12: Abschnitt
- 13: Peripherie
- 14.1, 14.2: Schweißnaht
- 15: Umschlagfalte
- 16: Stanzlinie
- 17; 18: Markierung
- 20: Absorptionskörper
- 21: Langloch
- 26: Tasche
- 30: Verpackungsbeutel
- 40: Sekundärverband

- 100: Primärverband

- A: Perforationsachse (v. 2)

## Patentansprüche

1. Flüssigkeitsdurchlässiger Primärverband (100) in Form eines schmiegsamen thermoplastischen Materialabschnittes (1.1; 1.2; 1,3: 1.4; 1.5), aufweisend:
- eine erste, glatte Oberfläche (4),
- eine der glatten. Oberfläche (4) abgewandte, zweite Oberfläche (5) des Materialabschnittes (1.1; 1.2; 1.3; 1.4; 1.5),
- eine Vielzahl von dreidimensionalen Perforationen (2'; 2' ; 21), deren Wände (3) von der ersten, glatten Oberfläche (4) beginnend jeweils in einen Randüberstand mit freier Kante (8) auslaufen und der zweiten Oberfläche (5) einen rauen Griff verleihen,
**dadurch gekennzeichnet, dass**
wenigstens eine der freien Kanten (8) in einen im Wesentlichen senkrecht zu einer Perforationsachse (A) abgewinkelten Abschnitt (12) übergeht.

2. Primärverband nach Anspruch 1, **dadurch gekennzeichnet, dass** die Wände (3) der Perforationen (2) im Wesentlichen konisch ausgebildet sind.

3. Primärverband nach Anspruch 1, **dadurch gekennzeichnet, dass** die Perforationen (2) entsprechend einer durchgängigen Meniskusbildung von Flüssigkeiten mit spezifischem Gewicht zwischen 0,9 und 1,2 ausgebildet sind.

4. Primärverband nach Anspruch 1, **dadurch gekennzeichnet, dass** die Perforationen (2) rund, oder oval sind oder in Form von länglichen Löchern vorliegen.

5. Primärverband nach Anspruch 1, **dadurch gekennzeichnet, dass** die Perforationen (2) pyramidenförmig sind und dass ihre Wände (3) segmentiert, d. h. voneinander mit Stanzlinien (16) getrennt sind.

6. Primärverband nach Anspruch 4, **dadurch, gekennzeichnet, dass** die runden Perforationen (2) einen Durchmesser von 650 bis 800 µm haben.

7. Primärverband nach Anspruch 4, **dadurch gekennzeichnet, dass** die länglichen Löcher 900 bis 1.200 µm lang sind bei einer Breite von etwa 700 µm.

8. Primärverband nach einem der vorhergehenden Ansprüche, **dadurch gekennzeichnet, dass** der Materialabschnitt an seiner Peripherie (13) auf seinen beiden Seiten, d. h. sowohl an der ersten, als auch an der zweiten Oberfläche (4; 5) glatt ist, wobei die zuerst taue Oberfläche (5) an der Peripherie (13) thermisch geglättet ist.

9. Primärverband nach einem der vorhergehenden Ansprüche, **dadurch gekennzeichnet, dass** am Materialabschnitt (1.4) Markierungen (17; 18) eingebracht sind.

10. Primärverband nach einem der vorhergehenden Ansprüche, **dadurch gekennzeichnet, dass** der Materialabschnitt (1.5) mit wenigstens einer Umschlagfalte (15) versehen ist.

11. Primärverband nach einem der vorhergehenden Anspruch, **dadurch gekennzeichnet, dass** der Materialabschnitt mit einem umhüllten Absorptionskörper (20) verbunden ist.

12. Primärverband nach einem der vorhergehenden Ansprüche, **dadurch gekennzeichnet, dass** der Materialabschnitt zu einer Tasche (26) geformt ist, in die ein Absorptionskörper einlegbar ist.

13. Primärverband nach einem der vorhergehenden Ansprüche, **dadurch gekennzeichnet, dass** der Materialabschnitt Träger für Pharmaka ist.

14. Verwendung des Primärverbandes gemäß dem Anspruch 1 zur Wundabeckung als sterilisierter Primärverband (100), auf den saugfähige Sekundärverbände (40) auflegbar sind.

15. Verwendung des Primärverbandes gemäß dem Anspruch 1 zur Kompressionstherapie bei chronischen Wunden oder zur Unterdrucktherapie.

## Claims

1. A liquid-permeable primary dressing (100) in the form of a flexible thermoplastic material section (1.1; 1.2; 1.3; 1.4; 1.5), comprising:
- a first smooth surface (4),
- a second surface (5) of the material section (1.1; 1.2; 1.3; 1.4; 1.5) facing away from the smooth surface (4),
- a plurality of three-dimensional perforations (2'; 2"; 21) whose walls (3), starting from the first smooth surface (4), run out into an edge projection with a free edge (8) and impart a rough grip to the second surface (5),
**characterized in that**
at least one of the free edges (8) merges into a section (12) bent substantially vertical to a perforation axis (A).

2. Primary dressing according to claim 1, **characterized in that** the walls (3) of the perforations (2) are designed substantially conically.

3. Primary dressing according to claim 1, **characterized in that** the perforations (2) are formed according to a permeable meniscus formation of liquids with a specific weight between 0.9 and 1.2.

4. Primary dressing according to claim 1, **characterized in that** the perforations (2) are round or oval or are present in the form of oblong holes.

5. Primary dressing according to claim 1, **characterized in that** the perforations (2) are pyramid-shaped and that their walls (3) are segmented, i.e., separated from each other with stamped lines (16).

6. Primary dressing according to claim 4, **characterized in that** the round perforations (2) have a diameter of 650 to 800 µm.

7. Primary dressing according to claim 4, **characterized in that** the oblong holes are 900 to 1200 µm long with a width of approximately 700 µm.

8. Primary dressing according to any one of the preceding claims, **characterized in that** the material section is smooth on its periphery (13) on its two sides, that is, on the first as well as on the second surface (4; 5), wherein the surface (5) that was raw at first is thermally smoothed on the periphery (13).

9. Primary dressing according to any one of the preceding claims, **characterized in that** markings (17; 18) are applied on the material section (1.4).

10. Primary dressing according to any one of the preceding claims, **characterized in that** the material section (1.5) is provided with at least one turned-back fold (15).

11. Primary dressing according to any one of the preceding claims, **characterized in that** the material section is connected to an encased absorption body (20).

12. Primary dressing according to any one of the preceding claims, **characterized in that** the material section is formed to an envelope (26) into which an absorption body can be placed.

13. Primary dressing according to any one of the preceding claims, **characterized in that** the material section is a carrier for pharmaceuticals.

14. Use of the primary dressing according to claim 1 for covering wounds as a sterilized primary dressing (100) onto which absorbent secondary dressings (40) can be placed.

15. Use of the primary dressing according to claim 1 for compression therapy for chronic wounds or for vacuum therapy.

## Revendications

1. Pansement primaire perméable aux liquides (100) sous la forme d'une section de matière thermoplastique souple (1.1 ; 1.2 ; 1.3 ; 1.4 ; 1.5), comprenant :
- une première surface lisse (4),
- une seconde surface (5), opposée à la surface lisse (4), de la section de matière (1.1 ; 1.2 ; 1.3 ; 1.4 ; 1.5),
- une pluralité de perforations tridimensionnelles (2' ; 2" ; 21), dont les parois (3) s'étendant depuis la première surface lisse (4) se terminent chacune par un rebord en saillie possédant un bord libre (8) et rendent la seconde surface (5) rugueuse au toucher,
**caractérisé en ce que** :
au moins l'un des bords libres (8) se prolonge en une section (12) coudée de manière sensiblement perpendiculaire à un axe de perforation (A).

2. Pansement primaire selon la revendication 1, **caractérisé en ce que** les parois (3) des perforations (2) sont de forme sensiblement conique.

3. Pansement primaire selon la revendication 1, **caractérisé en ce que** les perforations (2) ont une forme correspondant à une formation générale dans le ménisque de liquides ayant un poids spécifique compris entre 0,9 et 1,2.

4. Pansement primaire selon la revendication 1, **caractérisé en ce que** les perforations (2) sont rondes ou ovales ou se présentent sous la forme de trous oblongs.

5. Pansement primaire selon la revendication 1, **caractérisé en ce que** les perforations (2) sont de forme pyramidale et **en ce que** leurs parois (3) sont segmentées, c'est-à-dire séparées les unes des autres par des lignes de découpe (16).

6. Pansement primaire selon la revendication 4, **caractérisé en ce que** les perforations rondes (2) ont un diamètre compris entre 650 et 800 µm.

7. Pansement primaire selon la revendication 4, **caractérisé en ce que** les trous oblongs ont une longueur comprise entre 900 et 1 200 µm pour une largeur d'environ 700 µm.

8. Pansement primaire selon l'une quelconque des revendications précédentes, **caractérisé en ce que** la périphérie (13) de la section de matière est lisse sur ses deux côtés, c'est-à-dire aussi bien sur la première que sur la seconde surface (4 ; 5), la périphérie (13) de la surface au départ rugueuse (5) étant lissée thermiquement.

9. Pansement primaire selon l'une quelconque des revendications précédentes, **caractérisé en ce que** des marquages (17 ; 18) sont pratiqués sur la section de matière (1.4).

10. Pansement primaire selon l'une des revendications précédentes, **caractérisé en ce que** la section de matière (1.5) est pourvue d'au moins un repli (15).

11. Pansement primaire selon l'une quelconque des revendications précédentes, **caractérisé en ce que** la section de matière est reliée à un corps absorbant enveloppé (20).

12. Pansement primaire selon l'une quelconque des revendications précédentes, **caractérisé en ce que** la section de matière est façonnée en une poche (26), dans laquelle un corps absorbant peut être inséré.

13. Pansement primaire selon l'une quelconque des revendications précédentes, **caractérisé en ce que** la section de matière est un véhicule pour produits pharmaceutiques.

14. Utilisation du pansement primaire selon la revendication 1 pour la protection d'une blessure en tant que pansement primaire stérilisé (100), permettant l'application de pansements secondaires absorbants (40).

15. Utilisation du pansement primaire selon la revendication 1 pour une thérapie de compression en cas de blessures chroniques ou d'une thérapie par pression négative.
